# EUROPEAN PATENT SPECIFICATION

(11) **EP 1 267 981 B1**
(45) Date of publication and mention of the grant of the patent: **23.11.2005**
(21) Application number: 00921960.1
(22) Date of filing: 07.04.2000
(51) Int. Cl.: A61M 25/00, A61M 1/10, A61L 29/06

(54) **INTRA-AORTIC BALLOON CATHETER HAVING AN ULTRA-THIN STRETCH BLOW MOLDED BALLOON MEMBRANE AND MANUFACTURING METHOD THEREFOR**
INTRA-AORTALER BALLONKATHETER MIT ULTRAFEINER BALLONMEMBRAN HERGESTELLT DURCH STRECKBLASFORMEN UND DESSEN HERSTELLUNGSVERFAHREN
SONDE A BALLONNET INTRA-AORTIQUE COMPORTANT UNE MEMBRANE DU BALLONNET ULTRAFINE FABRIQUEE PAR SOUFFLAGE AVEC BI-ETIRAGE ET SON PROCEDE DE FABRICATION

(43) Date of publication of application: 02.01.2003
(73) Proprietor: DATASCOPE INVESTMENT CORP., Montvale, N.J. 07645 (US)
(72) Inventor: LAKSIN, Olga, Scotch Plains, NJ 07076 (US)
(74) Representative: Harrison, Michael Charles
(86) International application number: PCT/US2000/009474
(87) International publication number: WO 2001/076674

(56) References cited:
- WO-A-00/27461
- WO-A-98/20929
- WO-A-99/53986
- US-A- 4 276 874

## Description

### BACKGROUND OF THE INVENTION

### 1. Field of the Invention

The invention relates to an improved intra-aortic balloon catheter. More particularly, the invention relates to an intra-aortic balloon catheter having an ultra-thin stretch blow molded balloon membrane with improved abrasion resistance, fatigue life, and aneurization resistance.

### 2. Description of the Prior Art

Intra-aortic balloon (IAB) catheters are used in patients with left heart failure to augment the pumping action of the heart. The catheters, approximately 1 meter long, have an inflatable and deflatable balloon at the distal end. The catheter is typically inserted into the femoral artery and moved up the descending thoracic aorta until the distal tip of the balloon is positioned just below or distal to the left subclavian artery. The proximal end of the catheter remains outside of the patient's body. A passageway for inflating and deflating the balloon extends through the catheter and is connected at its proximal end to an external pump. The patient's central aortic pressure is used to time the balloon and the patient's ECG may be used to trigger balloon inflation in synchronous counterpulsation to the patient's heart beat.

Intra-aortic balloon therapy increases coronary artery perfusion, decreases the workload of the left ventricle, and allows healing of the injured myocardium. Ideally, the balloon should be inflating immediately after the aortic valve closes and deflating just prior to the onset of systole. When properly coordinated, the inflation of the balloon raises the patient's diastolic pressure, increasing the oxygen supply to the myocardium; and balloon deflation just prior to the onset of systole lowers the patient's diastolic pressure, reducing myocardial oxygen demand.

Intra-aortic balloon catheters may also have a central passageway or lumen which can be used to measure aortic pressure. In this dual lumen construction, the central lumen may also be used to accommodate a guide wire to facilitate placement of the catheter and to infuse fluids, or to do blood sampling.

Typical dual lumen intra-aortic balloon catheters have an outer, flexible, plastic tube, which serves as the inflating and deflating gas passageway, and a central tube therethrough formed of plastic tubing, stainless steel tubing, or wire coil embedded in plastic tubing. A polyurethane compound is used to form the balloon.

All IAB catheters have two opposing design considerations. On the one hand, it is desirable to make the outer diameter of the entire catheter as small as possible: to facilitate insertion of the catheter into the aorta, maximizing blood flow past the inserted catheter, and to allow for the use of a smaller sheath to further maximize distal flow. On the other hand, however, it is desirable to make the inner diameter of the outer tube as large as possible because a large gas path area is required to accomplish the rapid inflation and deflation of the balloon. As a result of these opposing design considerations there is a need for a smaller catheter with a larger gas path area.

One method of making the outer diameter of the wrapped balloon portion of the catheter as small as possible is to wrap the balloon in its deflated state as tightly as possible around the inner tube. Wrapping the balloon tightly, however, has posed a number of difficulties. First, it is difficult to wrap the balloon tightly because of the friction between contacting surfaces of the balloon. Second, contacting surfaces of a tightly wrapped balloon may stick upon initial inflation. Datascope Investment Corp.'s co-pending application WO 9921604A discloses a lubricous coating for the balloon membrane which solves the above mentioned problems. The coating allows the balloon membrane to be wrapped tightly more easily and prevents sticking of the balloon membrane upon initial inflation.

A second method of making the outer diameter of the wrapped balloon portion of the catheter as small as possible is to decrease the size of the inner tube. Datascope Investment Corp.'s co-pending application WO 9921604A also discloses an intra-aortic balloon catheter with an inner tube having a smaller diameter only in the portion enveloped by the balloon membrane.

Although the above two methods have substantially reduced the overall insertion size of the intra-aortic balloon catheter the need still exists for greater size reduction. Furthermore, the need also exists for a balloon membrane with improved abrasion resistance, fatigue life, and aneurization resistance. Currently, the method of manufacturing polyurethane balloon membranes is solvent casting. This casting method does not provide the formed membrane with ideal physical and mechanical properties. A solvent caste membrane with the basic mechanical properties necessary for balloon pumping, typically has a single wall thickness of 0.1016 to 0.1524 mm (4 to 6 mils) (a mil is equal to one thousandth of an inch) which leads to a relatively large wrapped diameter of the balloon membrane. A thin solvent caste polyurethane membrane is capable of being manufactured, however, such a membrane does not demonstrate the required abrasion resistance and fatigue life. Therefore, the need exists for an improved method of making a balloon membrane which will allow for a balloon membrane having a reduced thickness, and at the same, having improved mechanical properties, including an improved abrasion resistance, fatigue life, and aneurization resistance.

The present invention comprises an intra-aortic balloon catheter having a stretch blow molded balloon membrane. The balloon membrane is made from thermoplastic elastomeric and/or semicrystalline materials such as but not limited to polyurethane and polyetheramid. As discussed above, intra-aortic balloon membranes are generally solvent cast.

The process of stretch blow molding catheter balloon membranes is known in the art. However, intra-aortic balloons have been traditionally made by solvent casting because intra-aortic balloon membranes require special characteristics: they must be substantially nondistensible and have high abrasion resistance, fatigue life, and aneurization resistance. Stretch blow molding has been traditionally used for angioplasty balloon membranes. These balloons are generally made from PET, Nylon, or PEBAX materials. These materials achieve their high strength at least partially because of the crystallization formed in their microstructure during the initial stretching step of the tube and as a result of quickly cooling the tube to a temperature below the crystallization temperature of the tube material. Crystallization of the microstructure increases the strength of the balloon membrane, however, as the inventors of the present invention have discovered, it has a negative effect on the abrasion resistance and fatigue life of the balloon membrane. Given that angioplasty balloon/PTCA therapy is a short duration therapy, crystallization is generally not a problem. Actually, it is quite useful given that it enhances the strength of the balloon material. Intra-aortic balloon therapy, on the other hand, involves repetitive inflation and deflation of the balloon membrane over a longer period of time. Accordingly, it is known in the art that stretch blow molded balloons are not appropriate for intra-aortic balloon membranes, which require high strength as well as high abrasion resistance and fatigue life.

An example of a balloon catheter for carrying out angioplasty is given in document WO99/53986. The catheter includes a shaft having an outer tubular member and an inner tubular member. An inflatable balloon is disposed on a distal section of the shaft. During use, the balloon may typically be inflated at pressures between 620.53 to 1,965kPa (90 to 285psi) for stent deployment in a coronary artery. Certain polyurethane materials may used for the balloon membrane which may be formed by providing a tubular product which may be heated and then subjected to an expansion pressure.

The present invention overcomes the above described obstacle by relying on the increased strength of polyurethane resulting from the high orientation and molecular interaction of the polyurethane molecules along the longitudinal axis of the tube. Said orientation results from stretching the tube until substantially all stretchability is removed. Polyurethane, and the other materials listed in the present application, do not exhibit significant stress induced crystallization upon stretching. Accordingly, the inventors of the present invention have discovered a means to create a balloon membrane strong enough to endure intra-aortic balloon pumping therapy without creating crystallization microstructure, which they have discovered, is detrimental to the abrasion resistance and fatigue life of the balloon membrane.

U.S. Patent No. 5370618 to Leonhardt discloses a pulmonary artery balloon catheter comprising a catheter terminating in a blow molded polyurethane balloon membrane. Pulmonary artery catheters are generally used for blood pressure measurements. Upon insertion and placement of the catheter the balloon membrane is inflated, occluding the housing blood vessel, so as to create a measurable pressure differential on either side of the balloon membrane. In order to achieve complete occlusion of the housing blood vessel the pulmonary artery catheter balloon membrane is elastic so as to allow expansion of the membrane. This is in contrast to the balloon membrane of the present invention which is stretch blow molded and is specifically manufactured to substantially eliminate distensibility in the final product.

### SUMMARY OF THE INVENTION

Accordingly, it is an object of the invention to produce an ultra-thin intra-aortic balloon membrane with superior abrasion resistance and fatigue life.

It is another object of the invention to produce a method for manufacturing said ultra-thin intra-aortic balloon membrane.

The invention is an intra-aortic balloon catheter having an ultra-thin stretch blow molded balloon membrane. The balloon membrane is made from thermoplastic elastomeric and/or semicrystalline materials such as but not limited to polyurethane and polyetheramid.

The method according to the invention is defined in claim 1. Further preferred features thereof are defined in subclaims 2-9.

The intra-aortic balloon catheter of the invention is defined in claim 10. Further preferred features thereof are defined in claims 11-18.

To the accomplishment of the above and related objects the invention may be embodied in the form illustrated in the accompanying drawings. Attention is called to the fact, however, that the drawings are illustrative only. Variations are contemplated as being part of the invention, limited only by the scope of the claims.

### BRIEF DESCRIPTION OF THE DRAWINGS

In the drawings, like elements are depicted by like reference numerals. The drawings are briefly described as follows.

FIG 1 is longitudinal cross section of a prior art intra-aortic balloon catheter.

FIG 1A is a transverse cross section of the prior art intra-aortic balloon catheter taken along line 1A-1A.

FIG 2 is a longitudinal cross sectional view of a distal portion of the improved intra-aortic balloon catheter.

FIG 3 is a side view of a mold having a balloon shaped cavity used to manufacture the balloon membrane.

FIG 4 is a top view of the first half of the mold containing and securing a stretched tube used as a preform to create the balloon membrane.

### DETAILED DESCRIPTION OF THE PREFERRED EMBODIMENTS

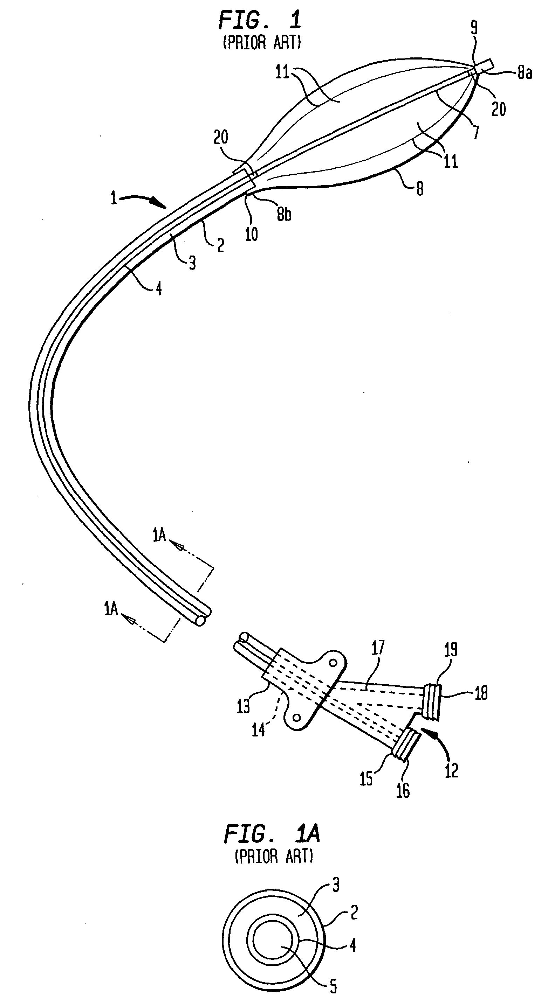

The general structure of an intra-aortic balloon catheter is best described in relation to FIGS 1 and 1A which illustrate a dual-lumen prior art intra-aortic balloon catheter. The catheter 1 is constructed of a plastic outer tube 2 forming a gas passageway lumen 3; and another plastic central tube 4 disposed within outer tube 2 and creating a central passageway or lumen 5 as may best be seen in FIG 1A.

A balloon 8 is disposed at the distal end of the catheter 1. The distal portion 7 of the central tube 4 extends beyond the distal end 10 of outer tube 2. The distal end 8A of the balloon 8 is attached to a tip 9 formed on the distal end 7 of central tube 4. The proximal end 8B of the balloon 8 is attached, by means of a lap joint, to the distal end 10 of the outer tube 2. The distal portion 7 of the central tube 4 supports the balloon 8. Said distal portion 7 must have sufficient strength to prevent inversion of the balloon 8 as it inflates and deflates under aortic pressure, but at the same time, be flexible enough to be safely inserted through an introducer sheath, moved through the arterial tree, and maintained in the thoracic aorta.

The balloon 8 is formed of a nonthrombogenic flexible material, such as polyurethane, and may have folds 11 formed as a result of wrapping the balloon 8 about the central tube 4 to ease insertion of the catheter 1. The balloon 8 has a single wall thickness of between 0.1016 to 0.1524 mm (4 to 6 mils). Radio-opaque bands 20 at the distal end of the catheter 1 aid in positioning the balloon 8 in the descending aorta.

Inflation and deflation of the balloon 8 is accomplished through the gas passageway lumen 3. The central passageway or lumen 5 can accommodate a guide wire for placement or repositioning of the catheter 1. When the guide wire is not disposed in the central passageway or lumen 5, the central passageway or lumen 5 may be used for measuring blood pressure in the descending aorta. This pressure measurement may be used to coordinate the inflation and deflation of the balloon 8 with the pumping of the heart, however, use of the patient's ECG is preferred. Additionally, the central passageway or lumen 5 may be used to infuse liquids into the descending aorta, or to sample blood.

At the proximal end 12 of the catheter 1 a hub 13 is formed on the proximal end 14 of the outer tube 2. The central passageway or lumen 5 extends through the hub 13 and a connector 16 is provided at the proximal end 15 (or exit) of the central passageway or lumen 5. Measurement of aortic pressure and blood sampling may be done through the proximal end 15 of the central passageway or lumen 5.

The proximal end 18 of the gas passageway or lumen 3 exits through a side arm 17 of the hub 13 on which is provided a connector 19. The proximal end 18 of the gas passageway or lumen 3 may be connected to an intra-aortic balloon pump.

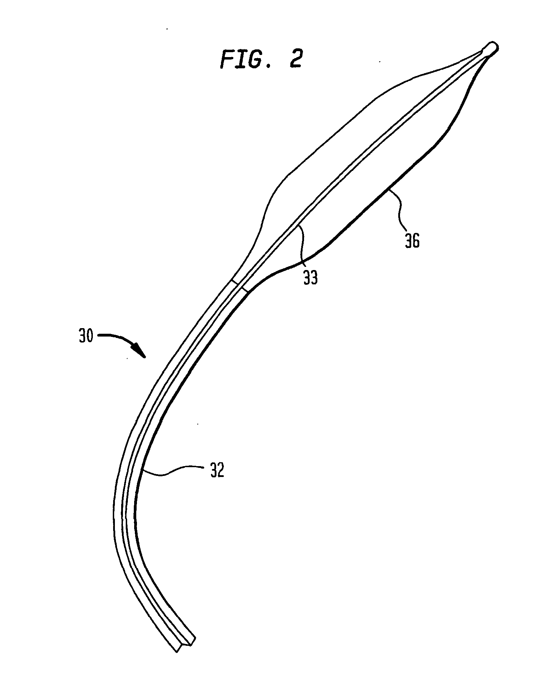

The present invention comprises an intra-aortic balloon catheter having an ultra-thin stretch blow molded balloon membrane. FIG 2 illustrates a longitudinal cross sectional view of a distal portion of an improved intra-aortic balloon catheter 30 of the present invention comprising an outer tube 32, an inner tube 33, a tip 34, and an ultra-thin polyurethane balloon membrane 36. The tip 34 is connected to a distal end of the balloon membrane 36. A distal end of the outer tube 32 is seamlessly welded to a proximal end of the balloon membrane 36. The balloon membrane 36 has a single wall thickness of between 0.0254 to 0.0508 mm (1 to 2 mils).

The balloon membrane 36 may be made from a variety of thermoplastic elastomeric and/or semicrystalline materials including but not limited to polyurethane and polyetheramid (known by its trade name as PEBAX, produced by ELF-Atochem of Europe).

Another feature of the invention involves the attachment of the outer tube 32 and the balloon membrane 36. The distal end of the outer tube 32 is seamlessly welded to the proximal end of the balloon membrane 36. The distal end of the outer tube 32 has the same inner and outer diameters as the proximal end of the balloon membrane 36, thus providing a smooth transition between the two parts without a constriction of the gas path. This is in contrast to the prior art catheter 1 of FIG 2 in which the proximal end of the balloon 8 and the distal end of the catheter 1 are attached by means of a lap joint. The lap joint is generally compressed during the manufacturing process in order to assure that the outer diameter of the lap joint matches that of the outer tube 2. Compression of the lap joint area leads to a restriction in the gas flow path. The present invention avoids this restriction through the use of a seamless weld.

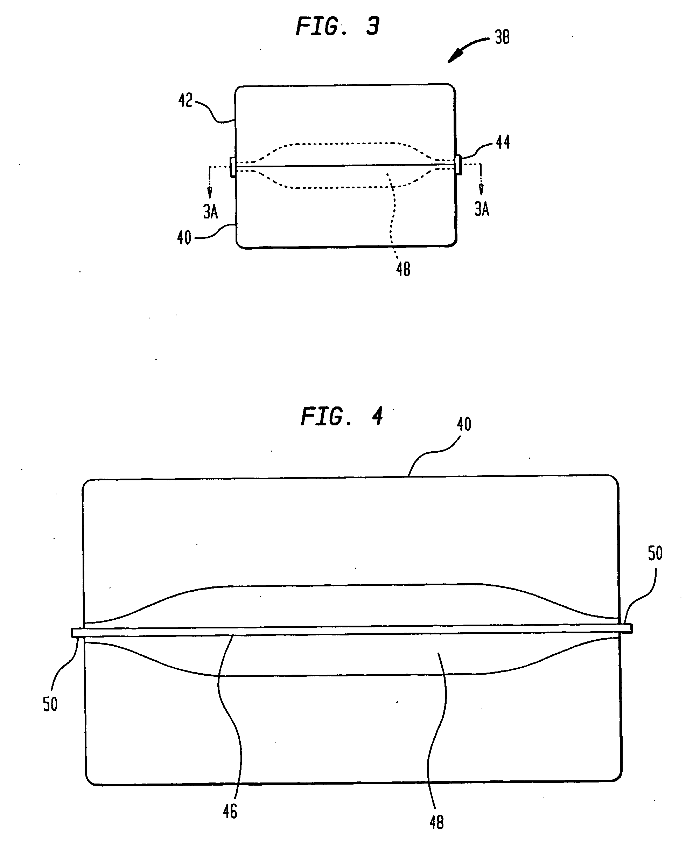

FIG 3 illustrates a side view of a mold 38 with tube clamps 44 on both sides for securing a tube 46 having ends 50 to the mold 38. The mold 38 has a first half 40 and a second half 42 and is utilized in the manufacturing of the ultra-thin balloon membrane 36 to assure the required profile of said balloon membrane 36. The first half of the mold 40 and the second half of the mold 42 together define a balloon shaped cavity 48 illustrated in FIG 3 as a shadowed segmented line.

The manufacturing process of the balloon membrane 36 comprises the following steps. First, a tube 46, as illustrated in FIG 4, is stretched to the desired length of the balloon membrane 36. In the preferred embodiment, and in this example, the tube 46 is 8 inches long, has a wall thickness of 0.508 mm (0.02 inches), and is made from polyurethane; however, the tube 46 may be of a different size and may made from other thermoplastic elastomeric and/or semicrystalline materials or other materials that do not display stress induced crystallization or a combination of such materials.

FIG 4 illustrates a top plain view of the first half 40 of the mold 38 which is identical to a view of the mold 38 taken along lines 3A-3A. The ends 50 of the tube 46 are secured by means of the clamps 44 (not shown in FIG 4 for clarity) to opposite sides of the mold 38. The second half 42 of the mold 38, as illustrated in FIG 3, is attached to the first half of the mold enclosing the stretched tube 46 in the balloon shaped cavity 48.

Stretching the tube 46, approximately two times its original length, prior to blowing orientates the polyurethane molecules along the longitudinal axis of the tube and substantially eliminates any further stretchability of the balloon membrane 36, at internal pressures of 13·79 to 27·58 kPa (two to four psi), by achieving a high molecular orientation and interaction. Note that this orientation and molecular interaction is achieved without significant crystallization; this is important to assure improved abrasion resistance and fatigue life. Significant crystallization is defined as any amount of crystallization which negatively affects the abrasion resistance and fatigue life of the balloon membrane. Polyurethane and polyetheramid, and other thermoplastic elastomeric and/or semicrystalline materials, do not exhibit stress induced crystallization. This is in contrast to the materials traditionally used for stretch blow molded angioplasty balloon catheter membranes which on average have approximately 30% crystallization.

The stretching step assures the substantial nondistensibility of the final balloon membrane 36 under balloon inflation pressures of approximately 13·79 to 27·58 kPa (2-4 psi). This is in contrast to the pulmonary artery balloon catheter, discussed above, whose polyurethane balloon membrane is specifically designed to be distensible so as to allow the membrane to expand, and thereby, occlude the blood vessel in which blood pressure is being measured.

The next manufacturing step involves filling the tube 46 with a gas at approximately 1,031·21 kPa (150 psi), i.e. blowing the tube 46. The amount of pressure applied depends on the grade of polyurethane: harder materials require higher pressure. In all cases, however, the pressure must be sufficient to force the tube 46 to take on the shape of the mold cavity 48. The tube 46 is then heated above the tube melting temperature while maintaining a pressure of 206·84-275·78 kPa (30-40 psi) in the tube 46. The final step involves quickly cooling the ballooned tube 46, to a temperature above the crystallization temperature of the tube 46, while maintaining a pressure of approximately 80 psi in the tube 46.

It should be noted that the above detailed manufacturing process is merely an illustrative example and will vary for different sized tubes and for tubes made of different materials. Manufacturing variables include tube material, tube length, tube thickness, tube diameter, the required pressure for blowing, the temperature for heating and cooling of the tube, and the amount of pressure maintained in the tube while heating and cooling the tube.

It should further be noted that balloon membrane of the present invention may be used with any variation of intra-aortic balloon catheters, including an intra-aortic balloon catheter having a co-lumen catheter, i.e. the inner lumen attached to or embedded in the catheter wall, or with any balloon catheter that requires a balloon membrane with improved abrasion resistance and fatigue life.

## Claims

1. A method for producing an ultra-thin balloon membrane having a wall thickness between 0.0254-0.0508 mm (between 1 to 2 units), for an intra-aortic balloon catheter (30) comprising the steps of:
a)stretching a tube (46) made from a thermoplastic elastomeric material to about two times its original length without creating significant crystallization until stretchability is eliminated under balloon inflation pressures of approximately 13·75 to 27·58 Kpa (2-4 psi); by achieving a high molecular orientation along the longititudinal axis of the tube;
b)blowing the tube (46) by increasing the pressure within the tube until the tube has sufficiently ballooned;
c)heating the ballooned tube; and
d)cooling the ballooned tube to above the crystallization temperature for the tube material.

2. The method as claimed in claim 1 wherein a pressure above ambient is maintained in the tube (46) while heating and cooling to maintain the ballooned shape of the tube.

3. The method as claimed in claim 1 wherein the tube (46) is at least partially composed of a thermoplastic elastomeric material.

4. The method as claimed in claim 1 wherein the tube (46) is at least partially composed of a semicrystalline material.

5. The method as claimed in claim 2 wherein the tube (46) is at least partially composed of a thermoplastic elastomeric semicrystalline material.

6. The method as claimed in claim 2 wherein the tube (46) is at least partially composed of polyurethane.

7. The method as claimed in claim 2 wherein the balloon membrane (36) is at least partially composed of polyetheramid.

8. The method as claimed in claim 2 wherein tube (46) is placed in a mold (38) having a cavity (48) therein prior to blowing.

9. The method as claimed in claim 2 wherein a pressure above ambient is maintained in the tube (46) while heating and cooling to maintain the ballooned shape of the tube.

10. An intra-aortic balloon catheter (30) comprising an outer tube (32) with proximal and distal ends, and a stretch blow molded balloon membrane (36) with a proximal end connected to the distal end of the outer tube, said balloon membrane having been stretched without creating significant crystallization to eliminate stretchability under balloon inflation pressures of approximately 13·79 to 27·58 kPa (2-4 psi), said membrane having a wall thickness between 0.0254-0.0508 mm (between 1 to 2 units) and whereby solid membrane has a high molecular orientation along its longitudinal axis.

11. The intra-aortic balloon catheter (30) as claimed in claim 10 wherein the balloon membrane (36) is at least partially composed of a thermoplastic elastomeric material.

12. The intra-aortic balloon catheter (30) as claimed in claim 10 wherein the balloon membrane (36) is at least partially composed of a semicrystalline material.

13. The intra-aortic balloon catheter (30) as claimed in claim 10 wherein the balloon membrane (36) is at least partially composed of a thermoplastic elastomeric semicrystalline material.

14. The intra-aortic balloon catheter (30) as claimed in claim 10 wherein the balloon membrane (36) is at least partially composed of polyurethane.

15. The intra-aortic balloon catheter (30) as claimed in claim 10 wherein the balloon membrane (36) is at least partially composed of polyetheramid.

16. The intra-aortic balloon catheter (30) as claimed in claim 11 wherein the balloon membrane (36) is made from polyurethane.

17. The intra-aortic balloon catheter (30) as claimed in any one of the preceding claims 10-16 wherein the intra-aortic balloon catheter further comprises an inner tube (33) at least partially disposed within an outer surface of the outer tube (32), a distal end of said inner tube being connected to a distal end of the balloon membrane (36).

18. The intra-aortic balloon catheter (30) as claimed in any one of the preceding claims 10-17, wherein said distal end of said outer tube (32) is seamlessly welded to a proximal end of said balloon membrane (36).

## Patentansprüche

1. Verfahren zur Herstellung einer extrem dünnen Ballonmembran mit einer Wanddicke zwischen 0,0254 - 0,0585 mm (zwischen 1 bis 2 mils) für einen intra-aortalen Ballonkatheter (30), das die Schritte umfasst:
a) Strecken einer aus einem thermoplastischen elastomeren Material hergestellten Röhre (46) bis zu etwa dem Zweifachen ihrer ursprünglichen Länge, ohne dabei bedeutsame Kristallisation zu erzeugen, bis die Verstreckbarkeit beseitigt ist, unter Ballonfülldrücken von näherungsweise 13,79 bis 27,58 kPa (2 - 4 psi), indem eine hohe molekulare Orientierung längs der Längsachse der Röhre erreicht wird;
b) Aufblasen der Röhre (46) durch Erhöhen des Drucks innerhalb der Röhre, bis sich die Röhre ausreichend aufgeblasen hat;
c) Erwärmen der aufgeblasenen Röhre; und
d) Abkühlen der aufgeblasenen Röhre auf oberhalb der Kristallisationstemperatur des Röhrenmaterials.

2. Verfahren nach Anspruch 1, bei dem ein Druck über Umgebungsdruck in der Röhre (46) beim Erwärmen und Abkühlen beibehalten wird, um die aufgeblasene Form der Röhre beizubehalten.

3. Verfahren nach Anspruch 1, bei dem die Röhre (46) wenigstens teilweise aus einem thermoplastischen elastomeren Material zusammengesetzt ist.

4. Verfahren nach Anspruch 1, bei dem die Röhre wenigstens teilweise aus einem semikristallinen Material zusammengesetzt ist.

5. Verfahren nach Anspruch 2, bei dem die Röhre (46) wenigstens teilweise aus einem thermoplastischen elastomeren semikristallinen Material zusammengesetzt ist.

6. Verfahren nach Anspruch 2, bei dem die Röhre (46) wenigstens teilweise aus Polyurethan zusammengesetzt ist.

7. Verfahren nach Anspruch 2, bei dem Ballonmembran (36) wenigstens teilweise aus Polyetheramid zusammengesetzt ist.

8. Verfahren nach Anspruch 2, bei dem die Röhre (46) vor dem Aufblasen in einer Form (38) angeordnet wird, die eine Kavität (48) aufweist.

9. Verfahren nach Anspruch 2, bei dem ein Druck über Umgebungsdruck in der Röhre (46) beim Erwärmen und Abkühlen beibehalten wird, um die aufgeblasene Form der Röhre beizubehalten.

10. Intra-aortaler Ballonkatheter (30), der eine Außenröhre (32) mit einem proximalen und einem distalen Ende und eine streckblasgeformte Ballonmembran (36) mit einem mit dem distalen Ende der Außenröhre verbundenen, proximalen Ende umfasst, wobei die Ballonmembran gestreckt worden ist, ohne bedeutsame Kristallisation zu erzeugen, um unter Ballonfülldrücken von näherungsweise 13,79 bis 27,58 kPa (2 - 4 psi) die Verstreckbarkeit zu beseitigen, wobei die Membran eine Wanddicke zwischen 0,0254 - 0,0508 mm (zwischen 1 bis 2 mils) aufweist, und die Membran eine hohe molekulare Orientierung längs ihrer Längsachse aufweist.

11. Intra-aortaler Ballonkatheter (30) nach Anspruch 10, bei dem die Ballonmembran (36) wenigstens teilweise aus einem thermoplastischen elastomeren Material zusammengesetzt ist.

12. Intra-aortaler Ballonkatheter (30) nach Anspruch 10, bei dem die Ballonmembran (36) wenigstens teilweise aus einem semikristallinen Material zusammengesetzt ist.

13. Intra-aortaler Ballonkatheter (30) nach Anspruch 10, bei dem die Ballonmembran (36) wenigstens teilweise aus einem thermoplastischen elastomeren semikristallinen Material zusammengesetzt ist.

14. Intra-aortaler Ballonkatheter (30) nach Anspruch 10, bei dem die Ballonmembran (36) wenigstens teilweise aus Polyurethan zusammengesetzt ist.

15. Intra-aortaler Ballonkatheter (30) nach Anspruch 10, bei dem die Ballonmembran (36) wenigstens teilweise aus Polyetheramid zusammengesetzt ist.

16. Intra-aortaler Ballonkatheter (30) nach Anspruch 11, bei dem die Ballonmembran (36) aus Polyurethan hergestellt ist.

17. Intra-aortaler Ballonkatheter (30) nach einem der vorherigen Ansprüche 10 bis 16, bei dem der intra-aortale Ballonkatheter ferner eine Innenröhre (33) umfasst, die wenigstens teilweise innerhalb einer Außenfläche der Außenröhre (32) angeordnet ist, wobei ein distales Ende der Innenröhre mit einem distalen Ende der Ballonmembran (36) verbunden ist.

18. Intra-aortaler Ballonkatheter (30) nach einem der vorherigen Ansprüche 10 bis 17, bei dem das distale Ende der Außenröhre (32) nahtlos mit einem proximalen Ende der Ballonmembran (36) verschweißt ist.

## Revendications

1. Procédé de fabrication d'une membrane de ballonnet ultrafine présentant une épaisseur de paroi située entre 0,0254 et 0,0508 mm (entre 1 et 2 mm), destinée à une sonde à ballonnet intra-aortique (30), comprenant les étapes consistant à :
a) étirer une tube (46) fabriqué dans un matériau en élastomère thermoplastique afin d'atteindre environ deux fois sa longueur d'origine sans former une cristallisation significative jusqu'à ce que l'étirabilité soit éliminée sous l'effet de pressions de gonflage du ballonnet d'approximativement 13,79 à 27,58 kPa (2 à 4 psi) en obtenant une forte orientation moléculaire le long de l'axe longitudinal du tube ;
b) souffler dans le tube (46) en augmentant la pression à l'intérieur du tube jusqu'à ce qu'il soit suffisamment gonflé ;
c) chauffer le tube gonflé ; et
d) refroidir le tube gonflé jusqu'à atteindre une température au-dessus de la température de cristallisation pour le matériau du tube.

2. Procédé selon la revendication 1, dans lequel une pression supérieure à la pression ambiante est maintenue dans le tube (46) pendant les étapes de chauffage et de refroidissement afin de maintenir la forme gonflée du tube.

3. Procédé selon la revendication 1, dans lequel le tube (46) est composé au moins partiellement d'un matériau en élastomère thermoplastique.

4. Procédé selon la revendication 1, dans lequel le tube (46) est composé au moins partiellement d'un matériau semicristallin.

5. Procédé selon la revendication 2, dans lequel le tube (46) est composé au moins partiellement d'un matériau semicristallin en élastomère thermoplastique.

6. Procédé selon la revendication 2, dans lequel le tube (46) est composé au moins partiellement de polyuréthane.

7. Procédé selon la revendication 2, dans lequel la membrane du ballonnet (36) est composée au moins partiellement de polyétheramide.

8. Procédé selon la revendication 2, dans lequel le tube (46) est placé dans un moule (38) présentant une cavité (48) à l'intérieur avant de procéder à l'étape du soufflage.

9. Procédé selon la revendication 2, dans lequel une pression supérieure à la pression ambiante est maintenue dans le tube (46) pendant les étapes de chauffage et de refroidissement afin de maintenir la forme gonflée du tube.

10. Sonde à ballonnet intra-aortique (30) comprenant un tube externe (32) avec des extrémités proximale et distale, et une membrane de ballonnet (36) moulée par soufflage-étirage avec une extrémité proximale reliée à l'extrémité distale du tube externe, ladite membrane de ballonnet ayant été étirée sans former de cristallisation significative afin d'éliminer l'étirabilité sous l'effet de pressions de gonflage du ballonnet d'approximativement 13,79 à 27,58 kPa (2 à 4 psi), ladite membrane présentant une épaisseur de paroi située entre 0,0254 et 0,0508 mm (entre 1 et 2 mm) et moyennant quoi ladite membrane présente une forte orientation moléculaire le long de son axe longitudinal.

11. Sonde à ballonnet intra-aortique (30) selon la revendication 10, dans laquelle la membrane du ballonnet (36) est composée au moins partiellement d'un matériau en élastomère thermoplastique.

12. Sonde à ballonnet intra-aortique (30) selon la revendication 10, dans laquelle la membrane du ballonnet (36) est composée au moins partiellement d'un matériau semicristallin.

13. Sonde à ballonnet intra-aortique (30) selon la revendication 10, dans laquelle la membrane du ballonnet (36) est composée au moins partiellement d'un matériau semicristallin en élastomère thermoplastique.

14. Sonde à ballonnet intra-aortique (30) selon la revendication 10, dans laquelle la membrane du ballonnet (36) est composée au moins partiellement de polyuréthane.

15. Sonde à ballonnet intra-aortique (30) selon la revendication 10, dans laquelle la membrane du ballonnet (36) est composée au moins partiellement de polyétheramide.

16. Sonde à ballonnet intra-aortique (30) selon la revendication 11, dans laquelle la membrane du ballonnet (36) est composée de polyuréthane.

17. Sonde à ballonnet intra-aortique (30) selon l'une quelconque des revendications 11 à 16, dans laquelle la sonde à ballonnet intra-aortique comprend en outre un tube interne (33) disposé au moins partiellement à l'intérieur d'une surface externe du tube externe (32), une extrémité distale dudit tube interne étant reliée à une extrémité distale de la membrane du ballonnet (36).

18. Sonde à ballonnet intra-aortique (30) selon l'une quelconque des revendications 11 à 17, dans laquelle ladite extrémité distale dudit tube externe (32) est soudée sans apport de matière à une extrémité proximale de ladite membrane du ballonnet (36).
